# EUROPÄISCHE PATENTSCHRIFT

(11) **EP 1 897 935 B1**
(45) Veröffentlichungstag und Bekanntmachung des Hinweises auf die Patenterteilung: **13.01.2010**
(21) Anmeldenummer: 07012742.8
(22) Anmeldetag: 25.01.2002
(51) Int. Cl.: C12M 1/00

(54) **Klimatisierter Lagerschrank**
Air-conditioned storage cabinet
Armoire de stockage climatisée

(30) Priorität: 26.01.2001 CH 136012001
(43) Veröffentlichungstag der Anmeldung: 12.03.2008
(62) Teilanmeldung aus: 02734885.3
(73) Patentinhaber: LICONIC AG, 9485 Nendeln (LI)
(72) Erfinder: Malin, Cosmas G., 9430 Mauren (FI)
(74) Vertreter: Sutter, Kurt

(56) Entgegenhaltungen:
- EP-A- 0 725 133
- WO-A-98/05753
- DE-U1- 20 004 202
- GB-A- 2 228 989
- JP-A- 2000 093 157
- US-A- 4 111 753
- US-A- 4 250 266
- US-A- 6 117 687
- US-B2- 6 673 595

## Beschreibung

Diese Anmeldung beansprucht die Priorität der Schweizer Patentanmeldung 0136/01, die am 26. Januar 2001 eingereicht wurde und deren ganze Offenbarung hiermit durch Bezug aufgenommen wird.

### Hintergrund

Die Erfindung betrifft einen Klimaschrank gemäss Oberbegriff des unabhängigen Anspruchs. Ein derartiger Klimaschrank ist in JP 2000-93157 offenbart. Ein anderer Klimaschrank ist in US 4 250 266 offenbart.

In der Substanzenforschung und Mikrobiologie müssen die verwendeten Objekte unter genau vorgegebenen Klimabedingungen gelagert werden. Die Temperaturen der Lagerung bewegen sich üblicherweise etwa vom Gefrierpunkt von Wasser bis hin zu Raumtemperatur. Die Objekte bestehen meist aus Behältern für mehrere Flüssigkeitsproben. Die Volumina der Proben werden stets kleiner und die Lagerlogistik der Objekte wird immer häufiger automatisch durchgeführt. Die Kapazität der Lagersysteme nimmt zu und es treten häufig mehrere solche Lagersysteme innerhalb eines Systems auf. Der Wert der in den Lagersystemen gelagerten Objekte ist äusserst gross und kann ein Vielfaches der Kosten eines gesamten Systems betragen. Eine Verwechslung von Objekten untereinander muss unter allen Umständen verhindert werden.

Es stehen heute Lagersysteme mit integrierter Umschlagvorrichtung zum Manipulieren der Objekte zur Verfügung. In der WO-98/05753 bzw. US 6 129 428 ist ein Klimaschrank mit einer automatischen Zugriffsmöglichkeit beschrieben. Es ist eine Benutzertüre und eine automatisch betätigbare Hilfstüre, sowie im Innern des Klimaschranks ein positionierbares Karussell mit einer Umschlagvorrichtung vorgesehen. Die integrierte Umschlagvorrichtung beinhaltet gezwungenermassen Sensoren und Antriebsmotoren. Beide geben Wärme an die Klimakammer ab. Wegen der hohen Isolierwerte der Schrankisolation verursachen bereits kleine Wärmequellen eine beträchtliche Eigenerwärmung der Klimakammer und unerwünschte Kondensation an den Wänden oder führen gar dazu, dass die Solltemperatur nicht mehr eingehalten werden kann. Die Objekte können bei dieser Einrichtung in einem Fehlerfall einfach und rasch durch die Verwendung von Lagerschächten be- und entladen werden. Allerdings ist die Lagerdichte pro Laborfläche bei diesem System klein. Ebenfalls nachteilig ist die Gestaltung der Lagerschächte, die kein fehlersicheres manuelles Be- und Entladen des Lagergutes erlaubt, nicht über die für automatischen Zugriffe notwendige mechanische Präzision aufweist und gar eine Verletzungsgefahr an den spitzen Kanten darstellen kann.

Eine andere Einrichtung verwendet Tablare zur Aufnahme der Objekte. Solche Ausführungen können grundsätzlich beliebig gross gebaut werden, haben aber den entscheidenden Nachteil, dass die Objekte nur umständlich und verbunden mit grossem Verwechslungsrisiko manuell be- oder entladen werden können.

In CH 690 645 ist ein Klimaschrank mit Benutzertüre und automatisch betätigbarer Hilfstüre beschrieben, wobei im Innern fest angeordnete Lagerschächte und eine Umschlagvorrichtung vorgesehen sind. Solche Anordnungen eigenen sich nur für eine kleine Anzahl von Lagerschächten und sind daher für den Einsatz grosser Lagersysteme nicht geeignet.

In der EP 1 155 743 ist eine Transporteinrichtung mit einem auf einer Schaufel angeordnetem Antrieb und einem Gegengewicht dargestellt. Diese Anordnung ist für eine sehr grosse Anzahl von Zugriffen nicht geeignet. Bei langen Verweildauern der Substanzen im Schrankinnern muss das Transportsystem jedoch wartungsfrei sein, da Wartungsarbeiten mit einem Verlust des Klimas verbunden ist. Nachteilig an der Vorrichtung dieser Offenlegungsschrift ist ferner, dass die Anordnung des Schaufelantriebs bei einer Verwendung des Schaufelmechnismus in Verbindung mit einem Karussell in einem Klimaschrank, eine Verschlechterung der Raumnutzung mit sich bringt, weil die liegende Anordnung des Schaufelantriebsmotors eine grosse seitliche Auslagerung der Zahnstange erfordert. Dies und die Baulänge des Schaufelantriebsmotors bedingen eine Vergrösserung des sich hinten und vorne verjüngenden Teils des Schaufelhalters. Bei der bei Karussellsystemen üblichen Anordnung des Schaufelmechnismus in einer Ecke der Klimakammer muss dadurch das Karussell weiter von der Ecke weggerückt werden, was wiederum eine Vergrösserung der Klimakammer nach sich zieht.

Den oben aufgeführten Einrichtungen ist gemeinsam, dass sie nebst der automatischen Zugriffsmöglichkeit eine Verbesserung der Klimastabilität durch die Verwendung einer kleinen Hilfstüre erzielen. Bei sehr häufigen Zugriffen in kurzen Zeitintervallen treten aber auch bei solchen Einrichtungen unerwünschte Klimaschwanken auf. Objekte, die der Klimakammer entnommen werden, brauchen zudem eine erhebliche Zeit, bis sie wieder die gewünschte Lagertemperatur erreichen. Dieser Nachteil ist besonders störend, da bei Lageranwendungen oft wiederholt ein gleiches Objekt innert kurzen zeitlichen Abständen der Klimakammer entnommen und wieder zugeführt wird. Auch genügen die bekannten Anordnungen nicht dem Bedürfnis nach hohen Zugriffsgeschwindigkeiten auch bei grossen Weglängen bei gleichzeitiger Wartungsfreiheit.

In zweifacher Hinsicht ist bei Lageranwendungen der Feuchte in der Klimakammer besondere Bedeutung beizumessen. Zum einen leidet sie unter Zugriffen weit stärker als beispielsweise die Temperatur, und zum anderen bestimmt sie die durch Verdunstung begrenzte Verweildauer der Objekte in der Klimakammer. Hierbei kann der Wunsch nach maximaler Feuchte - typischerweise bei wässerigen Lösungen - gleichermassen wie genaue, kontrollierte Feuchtigkeitswerte in Verbindung mit hygroskopischen Lösungen, wie beispielsweise DMSO, vorhanden sein.

Das Hinzufügen einer Kühlvorrichtung ist bei den betreffenden Anwendungen nicht ohne weiteres möglich, da das Kühlen in Verbindung mit hoher Feuchtigkeit problematisch ist. Zum Erzielen hoher Feuchtigkeit weisen Inkubatoren bekannter Art Isolationsplatten auf, die in einem bestimmten Abstand zur Inkubationskammer angeordnet sind und so einen Luftmantel um die Inkubationskammer bilden. Hierdurch wird eine gleichmässige Temperaturverteilung auf der Wandung der Inkubationskammer erreicht. Würde dieser Hohlraum gekühlt, würde Kondenswasser entstehen.

Bei einer für Kühlung geeigneten Isolation müsste der Hohlraum durch Schäumen gefüllt werden. Hierdurch würde jedoch die vom Hohlraum erzeugte Homogenisierung der Temperatur um den Nutzraummantel verloren gehen. Ferner bedingt das Schäumen aufwendige Lehren zur Aufnahme der Kräfte, die während dem Schäumvorgang auftreten. Schliesslich kann eine geschäumte Isolation kaum mehr geöffnet werden. Dies würde Wartungs- oder Reparaturarbeiten an. - um den Nutzraum angeordneten Komponenten - verunmöglichen oder zumindest sehr erschweren. Auch ist das Erstellen einer Lehre mit grossen Kosten verbunden. Bei den kurzen Produktlebenszeiten, kleinen Stückzahlen und der grossen Produktevielfalt im Markt der automatisierten Inkubatoren würden sich solche Investitionen nicht amortisieren. Handelsübliche Geräte besitzen nicht die gewünschten Dimensionen der Klimakammer. Auch würde die bei solchen Geräten übliche Kühlung, bei der ein Kühlelement im Nutzrauminneren oder in einem erweiterten Bereich des Nutzraums angeordnet wird, zu einem Austrocknen der Klimakammer führen. Dies ist dadurch bedingt, dass zum Erreichen einer Temperaturabsenkung die Temperatur des Kühlelementes deutlich unter die gewünschten Zieltemperatur gesenkt werden muss.

Die übliche Regelung der Temperatur bei Kühlgeräten bekannter Art geschieht durch Ein- und Ausschalten des Kühlaggregates. Um die Lebensdauer des Kühlaggregates auf einem akzeptablen Mass zu halten, muss hierbei in relativ grossen Zeitintervallen geschaltet werden. Dies hat zum Nachteil, dass relativ grosse Temperaturschwankungen auftreten. Weiter ist nachteilig, dass beim Ein- bzw. Ausschalten Erschütterungen auftreten, die sich störend auf die Präzision der Umschlagvorrichtung auswirken.

Bedingt durch die Wärmeentwicklung der Umschlagvorrichtung verschlechtert sich die erzielbare Klimaqualität. Klimaschwankungen und Erholzeiten, insbesondere bei einer raschen Folge von Zugriffen, sind erheblich. Einrichtungen obiger Art finden in aufwendigen und kostspieligen Systemen Anwendung, in die sie unter grossem Aufwand integriert werden müssen. Deren eingeschränkte Verwendbarkeit macht sich daher besonders nachteilig bemerkbar. Wegen der mangelnden Vielseitigkeit müssen daher je nach Anwendung mehrere Einrichtungen innerhalb eines Systems eingesetzt werden.

Wie die Feuchte reagiert die Begasung äusserst heftig auf Zugriffe. Selbst wenn der Zugriff über eine vergleichsweise kleine Öffnung geschieht, ist deren Öffnung mit einem deutlichen Einbruch der Begasungskonzentration verbunden. Die bekannten Vorrichtungen zur Messung der Gaskonzentration sind in der Klimakammer angeordnet und müssen den hohen Feuchtigkeitswerten widerstehen. Dadurch reduziert sich die Verwendbarkeit von Sensoren erheblich, und es müssen Sensoren eingesetzt werden, die nachteiliges Verhalten, wie niedrige Nutzsignale und Drift, aufweisen.

Schliesslich sind die bekannten Einrichtungen nur aufwendig in ein übergeordnetes System zu integrieren, da sehr viele Funktionen vom Integrator implementiert werden müssen und eine Vielzahl von Schnittstellen erforderlich sind oder gewisse Funktionen gar nicht vom übergeordneten System ausgeführt werden können oder gewisse Werte für dieses nicht zugänglich sind.

### Darstellung der Erfindung

Aufgabe der Erfindung ist es, zumindest einen Teil der oben erwähnten Nachteile zu bekämpfen. Insbesondere soll eine möglichst gute Regelung des Klimas erreicht werden.

Demgemäss wird ein Klimaschrank bereitgestellt, der eine gemeinsame Steuerung für die Klimatisiervorrichtung und die Umschlagvorrichtung aufweist. Dadurch können Synergien genutzt werden. Insbesondere kann eine gemeinsame Schnittstelle zur Verfügung gestellt werden. Ausserdem wird die Klimakontrolle auf die jeweiligen Prozessschritte der Umschlagvorrichtung abgestimmt, namentlich beim Öffnen der Hilfstüre der Klimakammer für den Umschlag von Objekten.

In einem bevorzugten Aspekt der Erfindung sind im Klimaschrank eine Heizvorrichtung und eine Kühlvorrichtung vorgesehen. Dadurch wird bessere Unabhängigkeit der Klimatemperatur von der Umgebungstemperatur und der Wärmeentwicklung der Umschlagvorrichtung erzielt. Insbesondere wird auch ein Verfahren zum Betrieb eines Klimaschranks bereitgestellt, bei welchem mit der Kühlvorrichtung mindestens ein Teil der von der Umschlagvorrichtung erzeugten Wärme aktiv entzogen wird, selbst wenn die Solltemperatur höher als die Umgebungstemperatur ist.

In einem weiteren bevorzugten Aspekt der Erfindung wird innerhalb oder ausserhalb der Klimakammer mindestens eine Temperaturanpassungs-Vorrichtung vorgesehen. Die Umschlagvorrichtung ist so ausgestaltet, dass sie ein ein- oder auszulagerndes Objekt vor Einbringen in den Lagerplatz oder nach Entnehmen aus dem Lagerplatz vorübergehend in Kontakt mit der Temperaturanpassungs-Vorrichtung bringen kann. Dies erlaubt es, die Temperatur des Objekts den jeweiligen Anforderungen schnell anzupassen.

Beim Einlagern von Objekten in den Klimaschrank kann die Temperaturanpassungs-Vorrichtung verwendet werden, um die Temperatur des Objekts der Kammertemperatur schnell anzupassen, ohne dass es dadurch zu Schwankungen der Kammertemperatur kommt. Ist die Temperatur in der Kammer höher als jene der Umgebung, so wird vorzugsweise eine geheizte Temperaturanpassungs-Vorichtung ausserhalb der Klimakammer vorgesehen, so dass die Objekttemperatur erhöht werden kann, bevor das Objekt in die Kammer eingeführt wird. Dadurch wird Kondensatbildung verhindert. Bei Kühlanwendungen wird vorzugsweise eine Temperaturanpassungs-Vorrichtung im Innern der Klimakammer verwendet, auf welcher die Objekttemperatur rasch abgesenkt werden kann.

In einem weiteren bevorzugten Aspekt der Erfindung wird der Klimaschrank mit einer Gerätezentriervorrichtung ausgestattet, welche eine selbstzentrierende Ankopplung an ein externes System bzw. eine externe Vorrichtung, wie z.B. ein externes Objektfördersystem, erlaubt. Dadurch wird der Aufbau und Umbau entsprechender Anlagen vereinfacht.

In einem anderen bevorzugten Aspekt der Erfindung sind in der Klimakammer mehrere, übereinander angeordnete Lagerschächte angeordnet. Dadurch verbessert sich die Raumnutzung in vertikaler Richtung. Dies führt zu einer Erhöhung der Lagerkapazitäten bei gleichzeitig gegebener einfacher manueller Beschickung. Alternativ oder zusätzlich hierzu können höhere Lagerschächte verwendet werden, die mit mindestens einem Versteifungselement verstärkt sind.

Ein weiterer bevorzugter Aspekt der Erfindung bezieht sich auf einen Lagerschacht aus Metall mit seitlichen Stegen, die Auflagen zur Aufnahme der Objekte bilden. Jeder Steg ist ein form- oder kraftschlüssig an einer der Seitenwände befestigtes Metallteil. Dadurch wird die Herstellung einfach und die Stege können dennoch exakt gefertigt werden. Dank der Vollmetall-Konstruktion bleibt der Lagerschacht autoklavierbar.

In einem anderen bevorzugten Aspekt sind die Wände des Klimaschranks aus Blechen hergestellt. Ein Teil der Wände weist abgebogene Kanten auf, an welchen die jeweils benachbarten Wände befestigt werden. Dadurch wird die Herstellung des Klimaschranks vereinfacht.

In einem weiteren bevorzugten Aspekt besitzt die Umschlagvorrichtung des Klimaschranks einen Schaufelhalter, der um eine vertikale Achse schwenkbar, sowie parallel zur vertikalen Achse verfahbar ist. Der Schaufelhalter trägt die Schaufel, die mit einem am Schaufelhalter befestigten Ausfahrantrieb senkrecht zur vertikalen Achse ausfahrbar ist. Der Ausfahrantrieb ist am hinteren Ende des Schaufelhalters angeordnet und die Längsachse des Ausfahrantriebs (624) steht vertikal. Dies hat den Vorteil, dass der Raum am hinteren Ende des Schaufelhalters gut ausgenutzt wird.

Ein anderer bevorzugten Aspekt der Erfindung bezieht sich auf Klimaschränke, in deren Klimakammer mindestens ein drehbar angeordnetes Karussell vorgesehen ist, in welchem eine Vielzahl Lagerplätzen zur Aufnahme der zu lagernden Objekte sternförmig um eine Karussellachse angeordnet ist. Die Umschlagvorrichtung besitzt eine Schaufel, welche horizontal und vertikal verfahrbar und um eine vertikale Achse schwenkbar ist. Zur Gewichtskompensation bei vertikalen Bewegungen der Schaufel ist ein über eine Kraftumlenkung befestiges Gegengewicht in der Karussellachse vorgesehen. Dadurch kann der Raum in der Karussellachse gut genutzt werden.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1a die Frontansicht einer ersten Ausführung des Klimaschrankes,
Fig. 1b die Frontansicht einer zweiten Ausführung des Klimaschrankes,
Fig. 2a die Frontansicht eines Ausführungsbeispiels eines Lagerschachtes,
Fig. 2b eine Seitenansicht auf den Lagerschacht nach Fig. 2a
Fig. 2c eine Detailansicht des Lagerschachtes nach Fig. 2a,
Fig. 2d ein Ausführungsbeispiel einer Versteifungsvorrichtung des Lagerschachtes,
Fig. 3a eine Schnittdarstellung einer erfindungsgemässen Isolation,
Fig. 3b eine Schnittdarstellung einer ersten Temperiervorrichtung mit Peltierelementen
Fig. 3c eine Schnittdarstellung einer zweiten Temperiervorrichtung mit Peltierelementen
Fig. 3d ein Blockschema zur erfindungsgemässen Kühlerregelung mit Kompressoraggregat,
Fig. 4a die Seitenansicht eines Ausführungsbeispiels einer Temperaturanpassungs-Vorrichtung,
Fig. 4b die Seitenansicht eines Ausführungsbeispiels eines Klimaschranks mit zwei Temperaturanpassungs-Vorrichtungen,
Fig. 5a die Seitenansicht eines Klimaschranks mit Gerätezentriervorrichtung,
Fig. 5b die Aufsicht des Klimaschranks mit Zentriervorrichtung,
Fig. 6a die Aufsicht eines Klimaschranks mit vorteilhaftem Schaufelantrieb,
Fig. 6b eine Aufsicht einer Umschlagvorrichtung mit Gegengewicht und
Fig. 7 ein Blockschema der Steuerung der erfindungsgemässen Einrichtung.

### Wege zur Ausführung der Erfindung

Gemeinsam allen hier dargestellten Ausführungen umfasst der erfindungsgemässe Klimaschrank eine Klimakammer 2 und eine Steuerkammer 4.

In Klimakammer 2 sind eine Vielzahl von Lagerplätzen 8 für die zu lagernden Objekte 9 vorgesehen. Sie besitzt eine äussere und eine innere Benutzertüre 210 bzw. 220 an der Vorderseite und eine Hilfstüre 15 an der Rückseite. Die Benutzertüren 210, 220 dienen dem Manuellen Zugriff auf das Innere der Klimakammer 2. Die äussere Benutzertüre 210 ist thermisch isoliert und undurchsichtig, die innere Benutzertüre besitzt eine transparente, thermisch isolierende Doppelverglasung. In der Klimakammer 2 ist eine Umschlagvorrichtung 620 zum automatischen Transport der Objekte zwischen den Lagerplätzen und einer Übergabeposition ausserhalb des Klimaschranks angeordnet, wobei hierzu die Hilfstüre 15 verwendet wird.

In Steuerkammer 4 sind eine Steuerung sowie verschiedene Aggregate 5 zur Erzeugung von Wärme, Kälte und/oder kontrollierter Atmosphäre angeordnet. Für die computergesteuerte Bedienung des Klimaschrankes ist eine Schnittstelle vorgesehen.

Das Innere der Klimakammer 2 wird von einer Klimatisiervorrichtung auf fester Temperatur und unter vorgegebenen atmosphärischen Bedingungen gehalten. Hierzu weist die Klimatisiervorrichtung die bereits erwähnten Aggregate zur Erzeugung von Wärme, Kälte und/oder kontrollierter Atmosphäre auf, sowie verschiedene im folgenden beschriebene Teile.

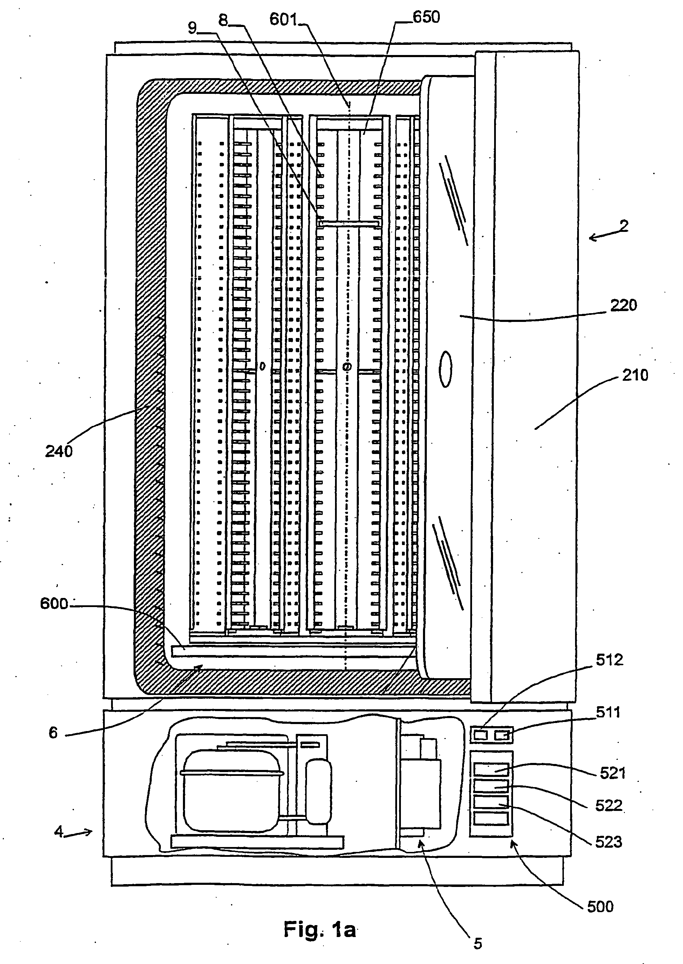

In Fig. 1a ist ein Ausführung des Klimaschrank abgebildet. Sie besitzt eine Anzeige 500 mit Anzeigefeldern 512 - 523 zum Anzeigen wichtiger Informationen, wie z.B. der Temperatur, relativen Feuchtigkeit und dem CO₂-Gehalt in Klimakammer 2. Ein akustisches Warnsystem 512 informiert den Benutzer über unzulässige Systemzustände. Zudem sind ein- oder mehrere Bedienelemente 511 vorgesehen, über die sich ein Teil der Funktionen des Klimaschrankes steuern lassen.

In der Ausführung nach Fig. 1a ist in Klimakammer 2 ein um eine vertikale Achse 601 drehbares Karussell 600 angeordnet. Auf dem Karussell 600 sind versteifte Lagerschächte 650 entnehmbar gelagert.

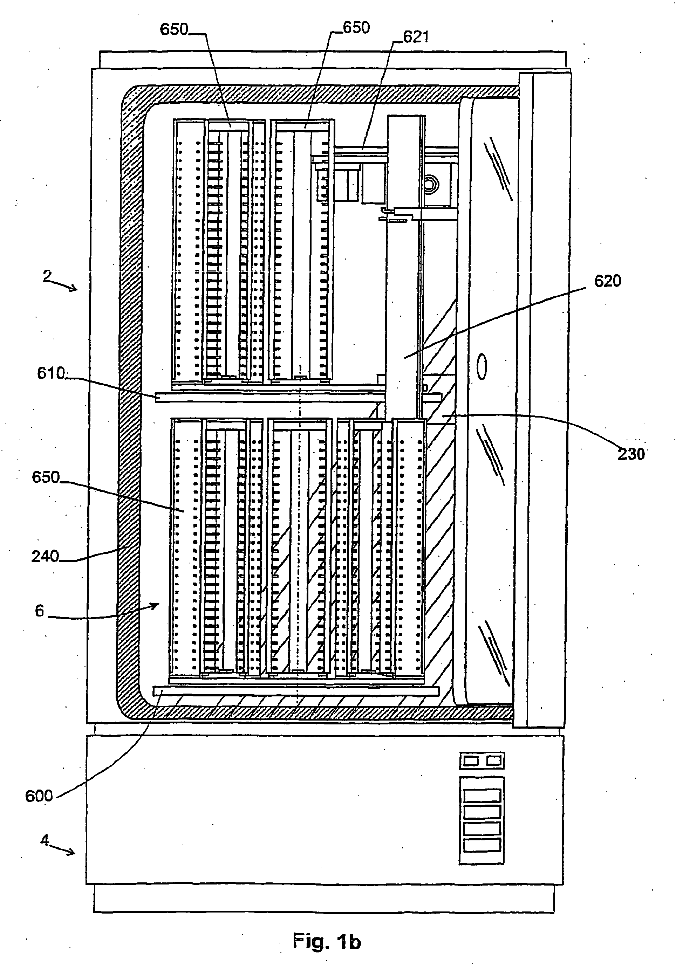

In Fig. 1b ist ein Klimaschrank mit einer Klimakammer 2 dargestellt, bei dem zur Vergrösserung der Lagerkapazität bei gleichbleibender Grundfläche mehrere Lagerschächte 650 bzw. zwei drehbare Karusselle 600, 610 übereinander angeordnet sind. Durch die Anordnung von zwei Karussellen übereinander kann auf eine Versteifung der Lagerschächte 650 auf den Karussellen 600, 610 verzichtet werden.

Die Karusselle der Klimaschränke nach Fig. 1a und 1b werden von einer (nur in Fig. 1b sichtbaren) Umschlagvorrichtung 620 be- und entladen. Umschlagvorrichtung 620 besitzt eine Schaufel 621 zur Aufnahme eines Objekts, wobei die Schaufel 621 um eine vertikale Drehachse gedreht, parallel zur Drehachse bewegt und senkrecht zur Drehachse ausgefahren werden kann. Eine derartige Umschlagvorrichtung ist in US 6 129 428 beschrieben.

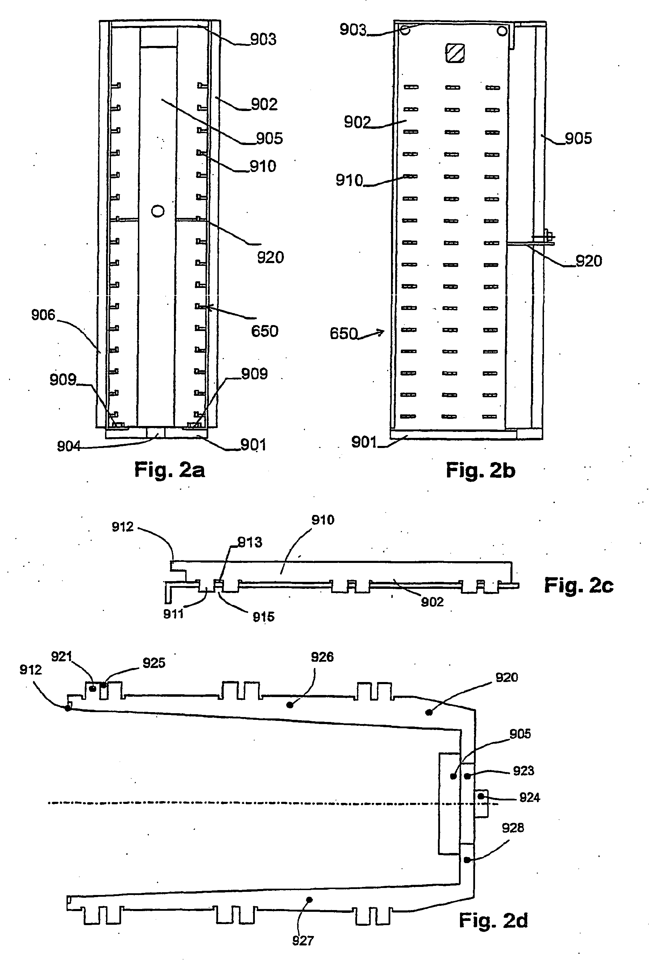

Fig. 2a und 2b zeigen ein Ausführungsbeispiel eines Lagerschachtes 650. Es sind eine linke und eine rechte Seitenwand 902, 906 aus Blech auf einer Grundplatte 901 mit einer Haltevorrichtung 904 angeordnet. Die Seitenwände 902, 906 sind an ihren oberen Enden über eine Griffplatte 903 verbunden. Zwischen Griffplatte 903 und Grundplatte 901 ist an der Rückseite des Lagerschachts ein Wandelement 905 als Verbindungselement angeordnet. An den Seitenwänden 902, 906 sind seitliche Stege 910 zur Aufnahme der Objekte angeordnet. Die Stege 910 sind Metallteile, die form- oder kraftschlüssig an einer der Seitenwände befestigt sind. Somit können sie separat und mit hoher Präzision gefertigt werden. Da sie aus Metall sind, können sie dennoch hohen Temperaturen (z.B. bei der Autoklavierung) standhalten.

Die Stege 910 sind vorzugsweise aus Blechen geformt und weisen nach oben abgebogene vordere Enden auf, die Rückhaltestifte 912 bilden und das Herausrutschen der Objekte verhindern.

Fig. 2c gibt eine Detailansicht eines Steges 910 wider. An der der Seitenwand 902 bzw. 906 des Lagerschachtes zugewandten Seite weist Steg 910 Spreizstücke 911 auf. Die Spreizstücke 911 sind durch Öffnungen 913 der Seitenwände 902, 906 geführt. Jedes Spreizstück 911 besteht aus zwei Laschen. Zwischen den Laschen 911 ist ein Spalt 915 angeordnet, über den sich die Laschen 911 mittels eines Werkzeuges spreizen lassen und so in den Öffnungen 913 der Seitenwände 902, 906 formschlüssig oder zumindest kraftschlüssig gehalten werden.

Zur Verbesserung der Stabilität liegen die Seitenwände 902, 906 mit abgewinkelten Enden 909 auf der Grundplatte 901 auf und sind über die Enden 909 mit der Grundplatte verschraubt oder in anderer Weise über die Enden 909 an der Grundplatte befestigt.

Da der Lageschacht 650 ein herausnehmbares Transportgebinde darstellt, dessen Eigengewicht klein sein soll, ist dieser aus relativ dünnem Blech gefertigt. Wegen der kleinen Materialstärken lassen sich die so aufgebauten Lagerschächte nur mit begrenzter Höhe in der erforderlichen Präzision fertigen. Aus diesem Grund kann zwischen dem oberen und dem unteren Ende des Lagerschachts wenigstens ein Versteifungselement 920 angeordnet werden.

Fig. 2d zeigt ein Ausführungsbeispiel des Versteifungselements 920. Es sorgt für eine exakte Positionierung der Seitenwände 902, 906. Analog zu den Stegen 910 verfügt das Versteifungselement 920 über Stegflächen 926, 927, die über einen Verbindungssteg 928, der hinter dem Wandelement 905 verläuft, verbunden sind. Der Verbindungssteg 928 ist am Wandelement 905 befestigt. Zur seitlichen Befestigung weist das Versteifungselement 920 analog zu den Stegen 910 seitliche Laschen 921 mit Spalten 925 auf, die in entsprechende Öffnungen der Seitenwände 902, 906 eingeführt und dort gespreizt werden.

Rückseitig ist am Vesteifungselement 920 eine Befestigungsplatte 923 angeordnet, die über eine Schraube 924 mit dem Wandelement 905 verbunden ist.

Durch die ausschliessliche Verwendung von Stahl und die dargestellte Anordnung der Elemente wird eine besonders hohe Langzeitstabilität und Schockbeständigkeit der Lagerschächte erzielt. Weiters widerstehen die Lagerschächte hohe Temperaturen, wie sie beim Autoklavierem auftreten.

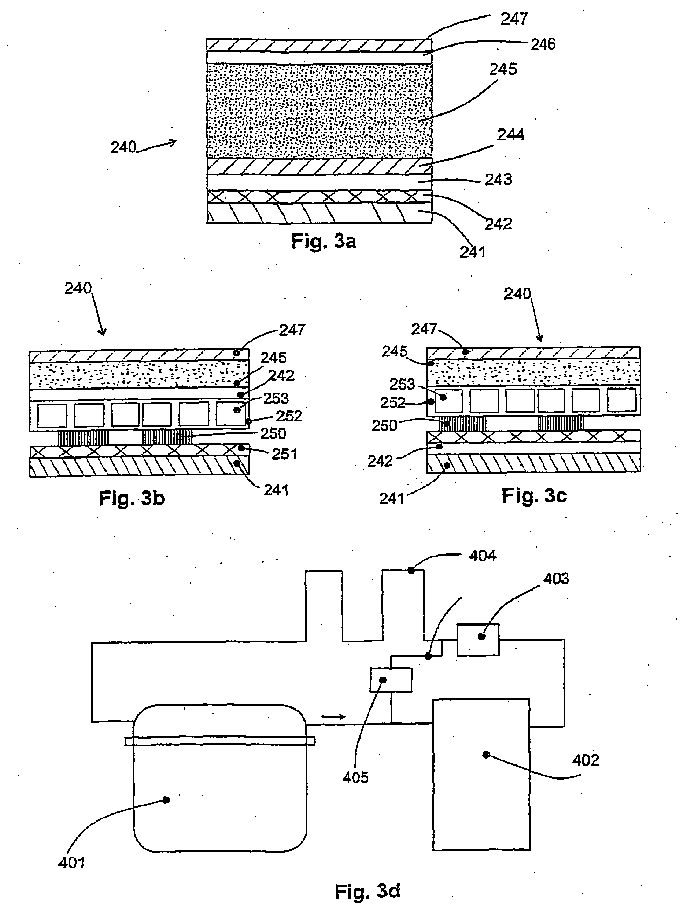

Fig. 3a zeigt den Aufbau einer Temperiervorrichtung 240, welche Teil der bereits erwähnten Klimatisiervorrichtung ist. Die Temperiervorrichtung 240 ist, mit Ausnahme der Türbereiche, um die ganze Klimakammer 2 angeordnet. Es sind eine Heizfläche 242 und eine Kühlfläche 244 um die Innenwand 241 der Klimakammer angeordnet. Die beiden Flächen sind, vom Innern der Klimakammer 2 her gesehen, als übereinander angeordnete Metallschichten ausgestaltet. Heizfläche 242 ist als Metallblech oder - folie mit aufgeklebten oder integrierten Heizdrähten ausgestaltet. Kühlfläche 244 ist ebenfalls ein Metallblech oder eine Metallfolie mit integrierten oder daran befestigten Rohren für eine Kühlflüssigkeit. Indem die Heizdrähte bzw. Rohre durch das Blech bzw. die Folie untereinander thermisch verbunden sind, wird die Temperaturverteilung homogenisiert, was unerwünschte Kondensatbildung an den Kammerwänden reduziert.

Zur weiteren Homogenisierung der Temperaturverteilung ist ein Luftspalt 243 vorgesehen. Da insbesondere die Rohre der Kühlfläche nicht beliebig fein und dicht angeordnet werden können, ist es von Vorteil, den Luftspalt 243 im Bereich zwischen Kühlfläche 244 und Innenwand 241 anzuordnen. In Fig. 3a ist Luftspalt 243 zwischen Kühlfläche 244 und Heizfläche 242 angeordnet, er kann jedoch auch zwischen Heizfläche 242 und Innenwand 241 vorgesehen sein.

Heizfläche 242 und Kühlfläche 244 sind Flächen, die wenigstens der Fläche des Klimakammermantels entsprechen und die Klimakammer 2 allseitig (mit Ausnahme der Bereiche der Türen) umgeben. Durch die flächige Gestaltung der Heiz- und der Kühlfläche wird eine möglichst grosse Fläche zur Temperatureinkopplung gebildet. Zudem wird hierdurch eine homogene Temperaturverteilung auf der Klimakammerwandung erzielt. Insbesondere ist von Vorteil, dass die Kühlvorrichtung über alle Wände der Klimakammer 2 angeordnet ist (gegebenenfalls mit Ausnahme der Bereiche der Benutzertüren.210, 220 und der Hilfstüre 15) und diese gleichmässig kühlt. So wird die Kondensatbildung reduziert und es kann hohe Feuchte erzielt werden.

Heizfläche 242, Kühlfläche 244 und Luftspalt 243 sind von einer Isolationsschicht 245 umgeben. Die Isolationsschicht ist gasdicht, so dass keine Luft von aussen zur Kühlfläche 244 vordringen kann. Indem die Kühlfläche 244 gegen aussen von einer gasdichten Isolationsschicht umgeben wird, kann eine unerwünschte Kondensatbildung verhindert werden.

Isolationsschicht 245 besteht aus gasdichten Isoliermatten, die auf Kühlfläche 244 aufgeklebt und an ihren Fugen gegeneinander abgedichtet sind. Das Verwenden von Matten ermöglicht den Aufbau einer gasdichten Isolation ohne schäumen zu müssen. Ausserdem können die Matten bei Bedarf entfernt werden, z.B. um Wartungsarbeiten an Kühlfläche 244 durchzuführen.

Zwischen der Aussenwand 247 des Klimaschranks und Isolationsschicht 245 kann zusätzlich eine wärmereflektierende Schicht 246 angeordnet werden.

Im Klimaschrank ist also sowohl eine Heizvorrichtung (beispielsweise in Form der Heizfläche 242), als auch eine Kühlvorrichtung (beispielsweise in Form der Kühlfläche 244) vorgesehen. Dies ist vorzugsweise auch dann der Fall, wenn das Innere des Klimakammer 2 wärmer als die Umgebung sein soll. Die Kühlvorrichtung erlaubt es, die Abwärme der Antriebsmotoren der Umschlagvorrichtung 620, die im Innern der Klimakammer 2 angeordnet sind, jederzeit aktiv abzuführen und so die Temperatur konstant zu halten. Somit ist die Verwendung der Kühlvorrichtung auch dann sinnvoll, wenn der Klimaschrank nur vorgesehen ist zum Lagern von Objekten bei, im Vergleich zur Umgebung, erhöhter Temperatur, z.B. bei 37°C. Die hier gezeigten Klimaschränke können jedoch ohne konstruktive Änderung auch zum Lagern von Objekten bei, im Vergleich zur Umgebung, reduzierter Temperatur verwendet werden, d.h. mit jedem Klimaschrank kann ein grosser Temperaturbereich von z.B. -20°C (oder tiefer) bis +70°C (oder höher) abgedeckt werden.

Das in Fig. 3b dargestellte Ausführungsbeispiel einer Temperiervorrichtung 240 verwendet Peltierelemente zum Kühlen bzw. Heizen. Die Peltierelemete 250 sind auf einem Thermoleitblech 251, das die Innenwand 241 der Klimakammer 2 umgibt, aufgebracht. Das Thermoleitblech dient wiederum der Homogenisierung der bezogen auf die gesamte Kühlfläche punktuellen Kühlung der Peltierelemente. Auf der zweiten Seite der Peltierelemente ist eine Wärmeanpassschicht 252 angeordnet. Im Falle der Kühlung des Thermoleitblechs 251 wird die Abwärme über die Wärmeanpassschicht 252 an die Umgebung abgeführt. Zur Verbesserung der Wärmeabfuhr weist die Wärmeanpassschicht 252 Hohlräume 253 auf, durch die ein Medium zur Wärmabfuhr strömt. Dieses Medium kann Luft oder eine Flüssigkeit sein. Die Wärmeanpassschicht 252 ist mit einer Heizschicht 242 versehen. Diese Heizschicht ist beim Heizen der Peltierelemente der Thermoleitschicht aktiv und verhindert ein Abkühlen der Wärmeanpassschicht 252 und ein damit verbundenes Bilden von Kondensation an der Wärmeanpassschicht. Die Isolationsschicht 245 verschliesst die Temperiereinrichtung gasdicht.

Bei der in der Fig. 3c dargestellten Schnittdarstellung einer Temperiervorrichtung 240 ist eine Heizschicht 242 zwischen der Wandung 241 und dem Peltierelement 250 angeordnet. Bei dieser Anordnung ist das Peltierelement 250 nur dann aktiv, wenn der Nutzraum gekühlt werden soll. Das Heizen des Nutzraums geschieht hier direkt über die Heizschicht 242.

Fig. 3d zeigt eine bevorzugte Ausführung eines Kühlaggregats zum Kühlen der Kühlfläche 244. Es besitzt - in an sich bekannter Art - einen Kompressor 401, der ein Kühlmedium zu einem Kondensor 402 befördert, von wo das Kühlmedium zu einem Einspritzventil 403 gelangt und von dort zu den eigentlichen Kühlrohren 404, welche der Kühlfläche 244 Wärme entziehen. Zusätzlich ist nun zwischen dem Ausgang des Kompressors 401 und dem Ausgang des Einspritzventils 403 ein Hilfsventil 405 angeordnet, mit dem Kondensor 402 (und Einspritzventil 403) überbrückt werden kann. Wird Hilfsventil 405 geöffnet, so wird die Temperatur des Kühlmediums in den Kühlrohren 404 erhöht.

Die Temperatur der Kühlrohre bzw. der Kühlfläche 244 wird vorzugsweise durch ein Öffnen und Schliessen des Hilfsventils 405 gesteuert, während Kompressor 401 dauernd in Betrieb ist. In Vergleich zu Geräten, bei denen zur Temperatursteuerung der Kompressor ein- und ausgeschaltet wird, werden bei dieser Lösung durch Reduktion der Ein- und Ausschaltzyklen des Kompressors Erschütterungen reduziert und die Lebensdauer der Anlage erhöht.

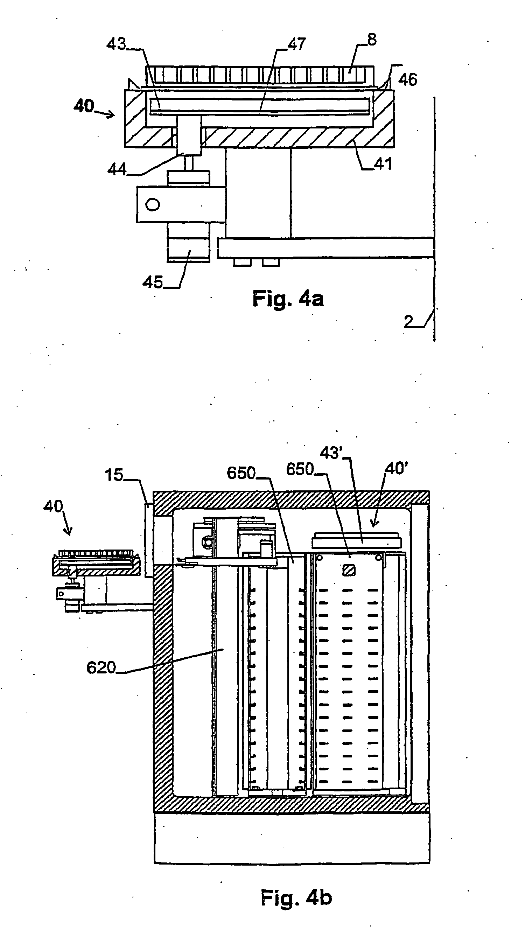

Fig. 4a stellt eine Seitenansicht einer Temperaturanpassungs-Vorrichtung 40 dar, wie sie z.B. vor der Hilfstüre 15 am Klimaschranks angeordnet werden kann. Ein einzulagerndes Objekt 8 wird auf einem Zentierrahmen 41 mit Zentrierstiften 46 angeordnet. Der Zentierrahmen 41 ist wannenförmig geformt. Innerhalb des Zentierrahmens ist eine temperierbare Platte 43 höhenverschiebbar angeordnet. Diese wird durch eine Temperaturquelle 47 gespiesen und lässt sich über einen Plattenlift 44 mit einem Antrieb 45 vertikal verschieben. Die Grösse von Platte 43 entspricht in etwa der Grösse eines einzulagernden Objektes 8. Durch Anheben von Platte 43 entsteht ein Kontakt zwischen Platte 43 und dem Boden des Objektes 8. Durch diesen Kontakt lässt sich die Geschwindigkeit der Temperatureinschwingzeit drastisch erhöhen. Durch Über- bzw. Untertemperatur der temperierbaren Platte lassen sich beinahe beliebig kurze Einschwingzeiten erzielen.

Soll ein Objekt 8 in den Klimaschrank eingebracht werden, kann es zuerst auf die Temperaturanpassungs-Vorrichtung 40 gebracht werden. Dort wird seine Temperatur der Innentemperatur des Klimaschranks angepasst oder zumindest angenähert. Sodann wird die Hilfstüre 15 geöffnet und die Umschlagvorrichtung 620 bringt das Objekt 8 an die gewünschte Position im Klimaschrank.

Durch die Temperaturanpassungs-Vorrichtung 40 ausserhalb des Klimaschranks können Temperaturschwankungen im Innern des Schranks, die durch die Wärmekapazität eingebrachter Objekte entstehen, reduziert werden.

Bei Heizanwendungen hat die Temperaturanpassungs-Vorrichtung 40 ausserhalb des Klimaschranks ferner den Vorteil, dass die Objekte beim Einbringen in den Schrank nicht wesentlich kälter sind als dessen Innenraum, so dass eine Kondensation vermieden wird.

Die Temperaturanpassungs-Vorrichtung 40 dient auch als Übergabeposition zwischen der Umschlagvorrichtung 620 und einem externen Transportsystem.

In Fig. 4b ist eine weitere Ausführung eines Schranks dargestellt, bei welcher die Lagerschächte 650 nicht auf einem Karussell, sondern stationär in der Klimakammer 2 angeordnet sind.

Beim Schrank nach Fig. 4b ist eine zweite Temperaturanpassungs-Vorrichtung 40' im Innern des Klimaschranks angeordnet. Diese weist eine metallene Auflageplatte 43' auf, welche sich entweder passiv der Temperatur des Schranks anpasst oder aktiv gewärmt oder gekühlt wird. Vorzugsweise besitzt sie ungefähr die gleiche Temperatur wie das Innere der Klimakammer 2.

Die im Innern angeordnete Temperaturanpassungs-Vorrichtung 40' wird vorzugsweise bei Kühlanwendungen eingesetzt. Ein in den Klimaschrank einzubringendes Objekt wird von der Umschlagvorrichtung 620 zuerst auf die Temperaturanpassungs-Vorrichtung 40` gelegt, wo seine Temperatur dank der Wärmekapazität der Auflageplatte 43' relativ rasch und weitgehend ohne Belastung der Temperaturstabilität der Kammer auf den Sollwert absinkt, ohne dass eine Kondensation stattfindet. Dabei dient die Auflageplatte 43' als Wärmereservoir zum Abfangen der Wärme des Objekts.

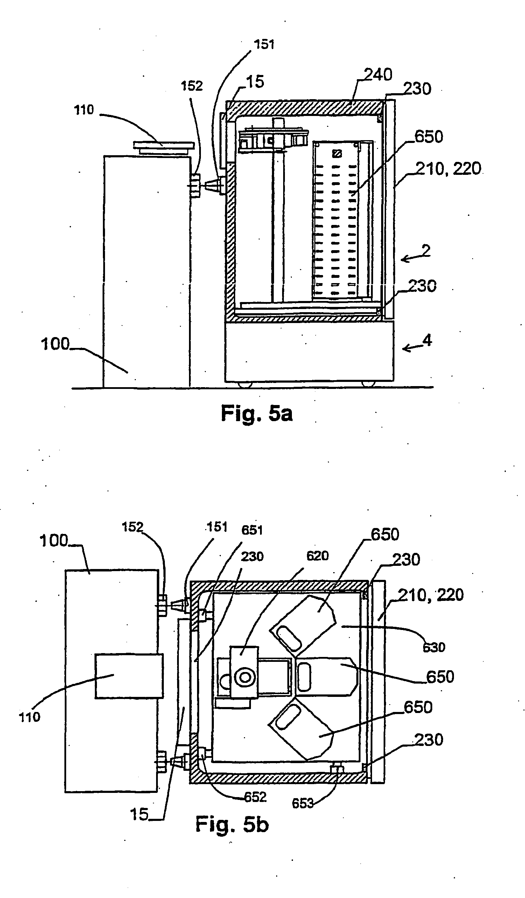

Fig. 5a und 5b zeigen eine weitere Ausführung eines Klimaschranks mit drei stationären Lagerschächten 650, die in symmetrischer Anordnung auf die Drehachse der Umschlagvorrichtung 620 ausgerichtet sind, wobei die Umschlagvorrichtung 620 zwischen den Lagerschächten 650 und der Hilfstüre angeordnet ist.

An der Rückseite des Klimaschrankes ist mindestens ein Konus 151 einer Gerätezentriervorrichtung vorgesehen. An dem umgebenden Objektfördersystem 100 ist ein Gegenstück 152 zur Gerätezentriervorrichtung befestigt. Mittels der Gerätezentriervorrichtung lässt der Klimaschrank einfach und exakt, in selbst zentrierender Weise zum umgebenden System ausrichten.

Wie aus Fig. 5b ersichtlich, sind die Umschlagvorrichtung 620 und die Lagerschächte 650 auf einer in der Klimakammer 2 angeordneten Grundplatte 630 aufgebaut. Die Grundplatte 630 ist in der Klimakammer 2 verschiebbar gelagert. Über einstellbare horizontale Anschläge 651, 652 und 653 lässt sich die Umschlagvorrichtung 620 mit den Lagerschächten 650 zur Klimakammer 2 positionieren, indem zwei Kanten der Grundplatte 630 gegen die Anschläge 651, 652 und 653 gedrückt werden. Die Anschläge 651, 652 und 653 sind so zur Gerätezentriervorrichtung angeordnet, dass die Lagerplätze, die Umschlagvorrichtung 620 und die Übergabeposition 110 zueinander in korrekter Beziehung stehen. Dies erlaubt eine schnelle und sichere Positionierung der Komponenten im Innern der Klimakammer 2 relativ zu den Konen 151 bzw. zum externen System 100.

Das externe Objektfördersystem 100 kann z.B. eine Übergabeposition 110 (beispielsweise in der Art der Temperaturanpassungs-Vorrichtung 40) sowie ein automatisiertes Transportsystem für die Objekte enthalten.

Wie aus Fig. 5a ersichtlich, ist der Klimaraum gegen die Benutzertüren 210, 220 mit einer Dichtung 230 abgedichtet. Vorzugsweise ist diese Dichtung 230 geheizt, z.B. durch einen im Dichtprofil verlegten Heizdraht, so dass es auch bei erhöhter Temperatur und feuchter Atmosphäre nicht zu einer Kondensatbildung bei der Dichtung im Innenraum kommt. Dadurch kann die maximale Feuchte im Innenraum erhöht werden.

Eine geheizte Dichtung 230 kann auch im Bereich der Hilfstüre 15 eingesetzt werden.

Um eine Kondensatbildung insbesondere bei Heizanwendungen an der Innenseite der inneren Benutzertüre 220 zu verhindern, kann die innere Benutzertüre geheizt werden. Dies kann z.B. durch Heizdrähte auf der Aussenseite der inneren Benutzertüre 220 oder zwischen der inneren und der äusseren Benutzertüre oder in der äusseren Benutzertüre erreicht werden.

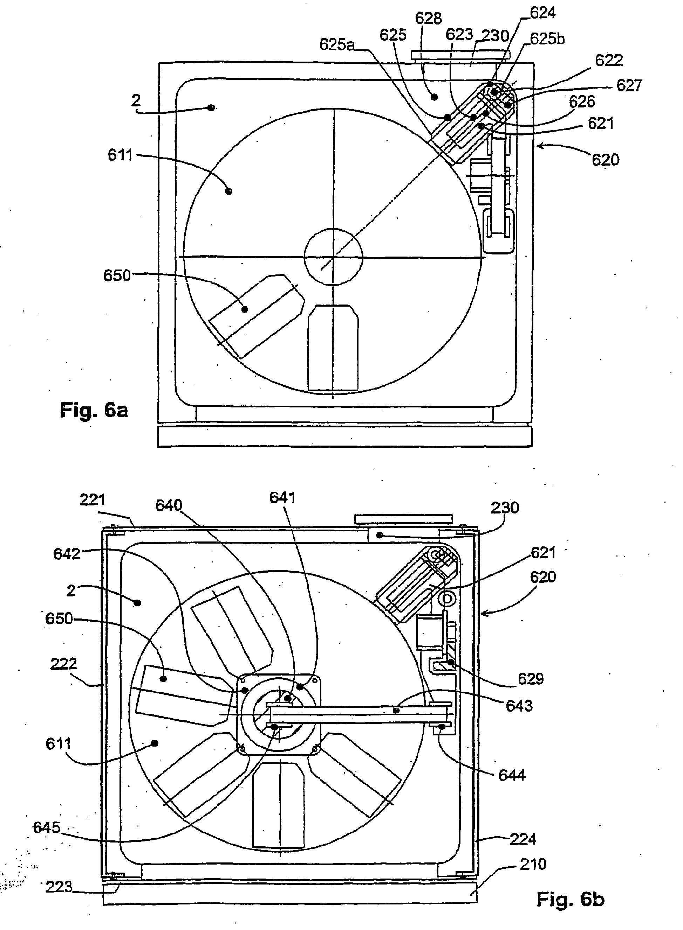

Fig. 6a zeigt einen Klimaschrank mit Karussell 611. Wie gezeigt, besitzt die Umschlagvorrichtung 620 einen vertikal beweglichen und um eine vertikale Achse drehbaren Schaufelhalter 625, der in einer Ecke der Klimakammer 2 angeordnet ist. Der Schaufelhalter 625 trägt die Schaufel 621. Er besitzt ein vorderes Ende 625a, über welches die Schaufel 621 ausfahrbar ist, und ein hinteres Ende 625b.

Die Schaufel 621 ist über ein Ritzel 622, das auf einer Zahnstange 623 läuft, horizontal angetrieben. Der Ausfahrantrieb 624 zum Ausfahren der Schaufel ist vertikal mit der Schaufel verfahrbar. Er ist mit seiner Längsachse senkrecht zur Schaufel 621 am hinteren Ende 625b des Schaufelhalters 625 montiert. Die Zahnstange 623 ist in einem kleinen Abstand von der Linearführung 626 der Schaufel 621 angeordnet. Damit ist der Schaufelantriebsmotor 624 platzsparend auf dem sich nach hinten verjüngenden Wagenteil 627 des Schaufelhalters angeordnet. Die Baulänge des Schaufelhalters 625 ist bei einem Klimaschrank mit Karussell von besonderer Bedeutung, da hier die Liftbaugruppe der Umschlagvorrichtung 620 in dem durch zwei Seitenwände der Klimakammer 2 und den Umkreis des Karussells 611 gebildeten dreieckähnlichen Raum 628 angeordnet ist. Der in diesem dreieckähnlichen Raum 628 liegende Teil der Diagonale der Klimakammer entspricht bei den gängigen Lagersystemen jedoch nur der Länge der Objekte.

Fig. 6b zeigt ein Lagersystem, das für häufige Zugriffe und zum raschen Zurücklegen der relativ grossen Weglängen der Umschlagvorrichtung 620 entlang ihrer vertikalen Führungsschiene 629 geeignet ist. Bei diesem Ausführungsbeispiel ist das Eigengewicht des vertikal beweglichen Teils der Umschlagvorrichtung 620 mit einem Gegengewicht 640 kompensiert. Zur optimalen Raumnutzung ist das Gegengewicht 640 im Drehzentrum des Karussells 611, d.h. in der Karussellachse, angeordnet. Das Gegengewicht 640 umgibt ein schützender Rohrmantel 641, der gleichzeitig als Luftführungsrohr genutzt wird. Dazu ist zusätzlich ein Gebläse 642 am Endteil des Rohrmantels 641 angeordnet. Das Gegengewicht ist an einem als Kraftumlenkung dienenden Flachriemen 643 aufgehängt, der über zwei Umlenkrollen 644, 645 mit dem vertikal beweglichen Teil der Umschlagvorrichtung verbunden ist. Durch Verwendung des Flachriemens 643 wird die Ermüdung der wegen den kleinen Radien der Umlenkrollenrollen 644, 645 erheblichen Beanspruchung des Riemens reduziert. Beim Flachriemen 643 ist dessen Breite gross gegen dessen Stärke.

Fig. 6b zeigt weiters die vorteilhafte Anordnung der Gehäuseteile, die den Anforderungen bezüglich Flexibilität und Einfachheit genügen. Es ist ein Frontblech 223 direkt über Isolationsteile mit dem Nutzraum verbunden. Am Frontblech 223 sind Seitenwände 222, 224 befestigt. Eine Rückwand 221 ist wiederum an den Seitenwänden 222, 224 befestigt. Die Seitenwände 222, 224 sind Bleche, welche an ihren Kanten im rechten Winkel abgebogen sind. Auf den gebogenen Flächen sind Gewinde vorgesehen. Mittels Schrauben wird das Frontblech und die Rückwand an den Gewinden befestigt. Für das Gehäuse werden somit lediglich einfache, zum Teil abgekantete Bleche benötigt.

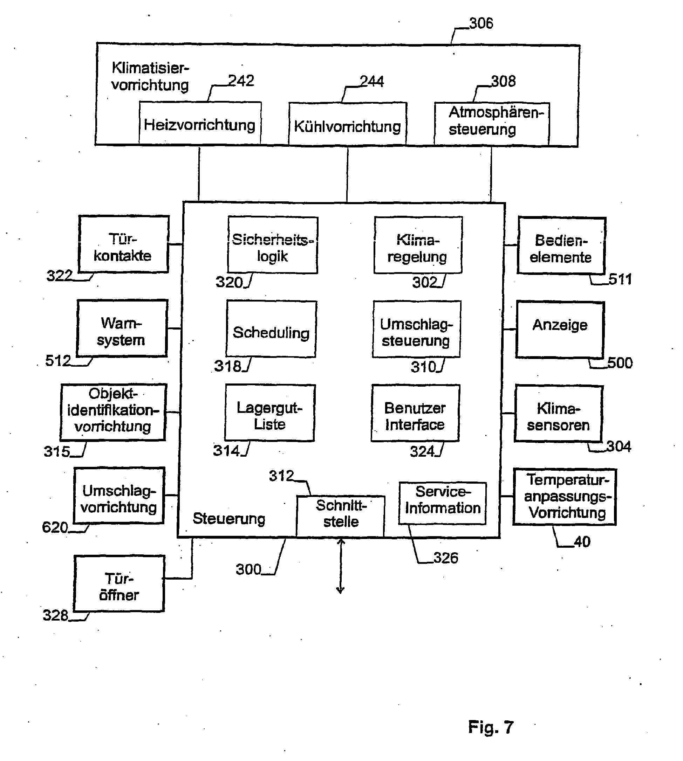

Fig. 7 zeigt wichtige Funktionsblöcke des Klimaschranks und insbesondere der Steuerung 300 desselben. Die Steuerung 300 basiert auf einem Rechner und besitzt verschiedene Funktionsteile, von denen die wichtigen in Fig. 7 dargestellt sind.

Eine Klimaregelung 302 kommuniziert mit Klimasensoren 304, die die Temperatur und Atmosphäre (z.B. Feuchtigkeit und CO₂-Konzentration) in der Klimakammer 2 messen. Entsprechend den Sensorsignalen und vorgegebenen Sollwerten steuert sie die Klimatisiervorrichtung 306, die die bereits erwähnte Heiz- und Kühlvorrichtung sowie eine Atmosphärensteuerung 308 aufweist. Die Atmosphärensteuerung 308 ist ausgestaltet, um der Klimakammer 2 z.B. ein Gas (z.B. CO₂ oder O₂) und/oder Wasser bzw. Dampf zuzuführen.

Die Klimatisiervorrichtung 306 kann auch weitere oder andere Parameter des Klimaraums steuern, wie z.B. eine Beleuchtung des Lagerguts mit Licht eines vorgegebenen Spektralbereichs.

Eine Umschlagsteuerung 310 steuert die Bewegungen der Umschlagvorrichtung 620, einen Türöffner 328 der Hilfstüre 15 und die Funktion der Temperaturanpassungs-Vorrichtung 40. Hierzu kann sie z.B. Befehlen folgen, die über eine Schnittstelle 312 von einem Host-System geliefert werden. Sie kann aber, zumindest teilweise, auch autonom arbeiten und z.B. selbst bestimmen, an welchem freien Lagerplatz ein einzulagerndes Objekt abgelegt werden soll.

Eine Lagergutliste 314 kann vorgesehen sein um festzuhalten, an welchen Lagerplätzen welche Objekte abgelegt sind. Hierzu kann am Klimaschrank auch eine Objektidentifikations-Vorrichtung 315 vorgesehen sein, die in der Lage ist, Objekte z.B. an der Übergabeposition 110 zu identifizieren. Die Objekt-Identifikation kann z.B. als Barcode-Leser oder Leser für elektronische Tags ausgebildet sein, wobei die Objekte mit einer entsprechenden Markierung versehen sind.

Eine Scheduling-Funktion 318 überwacht die Verweildauer jedes Objekts im Lagerschrank und vergleicht diese mit vorgegebenen Grenzwerten. Ist ein Objekt z.B. eine gewisse Zeit im Lagerschrank, so kann sie einen Alarm ausgeben, z.B. über Warnsystem 512 oder Schnittstelle 312.

Eine Sicherheitslogik 320 ist vorgesehen um Grenzwerte zu überwachen und gegebenenfalls einen Alarm auszulösen, und/oder um ein Öffnen der Benutzertüre 210 festzustellen und geeignete Massnahmen (Alarm, Benachrichtigung der Klimasteuerung) zu ergreifen. Hierzu sind Türkontakte 322 vorgesehen.

Ein mit Benutzer-Interface 324 gekennzeichneter Funktionsblock übernimmt die Steuerung und Überwachung der Anzeige 500 und der Bedienelemente 511.

Schnittstelle 312 stellt ein gemeinsames Interface für im wesentlichen alle Funktionen der Steuerung 300 zur Verfügung. Insbesondere können über Schnittstelle 312 die Sollwerte der Klimaregelung 302 gesetzt, die Operationen der Umschlagsteuerung 310 festgelegt, und die momentanen Zustandswerte des Klimaschranks abgefragt werden.

In der Steuerung 300 sind schliesslich Service-Informationen 326 abgelegt, wie z.B. die akkumulierte Betriebsdauer, die vom Benutzer oder über die Schnittstelle 312 ausgelesen werden können.

Indem alle Funktionen des Klimaschranks durch eine gemeinsame Steuerung kontrolliert werden, können vorteilhafte Synergien erzielt werden.

Beispielsweise weiss die Steuerung 300, wann die Hilfstüre 15 geöffnet wird, und wie lange sie voraussichtlich geöffnet sein wird. Sie kann diese Information dazu verwenden, die Klimaregelung 302 zu optimieren. Da beim Öffnen der Hilfstüre ein unerwünschter Wärmeübertrag zwischen Umgebung und Innerem der Klimakammer 2 stattfindet, kann beim Öffnen (z.B. bereits vor dem Öffnen, während der Öffnungsdauer und/oder nach dem Schliessen) ein vorübergehender Heiz- bzw. Kühlschub erzeugt werden. Auch kann ein Verlust von Feuchte und/oder Gas durch die geöffnete Türe instantan korrigiert werden. Es ist nicht notwendig, auf entsprechende Signale der Sensoren 304 zu warten. Die Steuerung 300 ist also so ausgestaltet, dass sie bei Öffnung der Hilfstüre 15 und ohne Abwarten einer Signaländerung der Klimasensoren 304 die Klimatisiervorrichtung 306 so steuert, dass einem Wärme- und/oder Gasaustausch durch die geöffnete Hilfstüre 15 entgegengewirkt wird.

Die Steuerung umfasst weiters einen Standby-Modus, in dem die aktiven Bauteile am Transportsystem mit reduzierter Leistung betrieben werden, wenn sich das Transportsystem nicht bewegt. Dadurch werden Störungen auf das empfindliche Klima in der Klimakammer reduziert.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Klimaschrank umfassend
eine Klimakammer (2),
eine Klimatisiervorrichtung (306) zum Erzeugen einer vorgegebenen Temperatur und Atmosphäre in der Klimakammer (2),
eine Vielzahl von in der Klimakammer (2) angeordneten Lagerplätzen (8) zur Aufnahme von zu lagernden Objekten,
eine in der Klimakammer (2) angeordnete Umschlagvorrichtung (620) zum Manipulieren der Objekte,
eine gemeinsame Steuerung (300) für die Klimatisiervorrichtung (306) und die Umschlagvorrichtung (620),
eine Hilfstüre (15), welche von der Steuerung (300) automatisch öffenbar ist und durch welche Objekte in die oder aus der Klimakammer transportierbar sind,
wobei der Klimaschrank eine Objektidentifizierungs-Vorrichtung (315) zum Auslesen von an den Objekten angebrachten Marken aufweist,
**dadurch gekennzeichnet, dass** die Steuerung dazu ausgestaltet ist, bei Öffnung der Hilfstüre (15) und ohne Abwarten einer Signaländerung allfälliger Klimasensoren die Klimatisiervorrichtung (306) derart zu steuern, dass einem Wärme- und/oder Gasaustausch durch die geöffnete Hilfstüre entgegengewirkt wird.

2. Klimaschrank nach Anspruch 1, wobei die Steuerung eine Schnittstelle (312) für ein Host-System aufweist, über welche sowohl die Klimatisiervorrichtung (306) als auch die Umschlagvorrichtung (620) steuerbar sind.

3. Klimaschrank nach einem der vorangehenden Ansprüche, **gekennzeichnet durch** eine Gerätezentriervorrichtung (151) ausgestaltet zur selbstzentrierenden Ankopplung an ein externes System, insbesondere ein externes Objektfördersystem

4. Klimaschrank nach einem der vorangehenden Ansprüche, wobei die Steuerung (300) ausgestaltet ist zum Überwachen der Verweildauer jedes Objekts in der Klimakammer (2).

5. Klimaschrank nach einem der vorangehenden Ansprüche, wobei die Steuerung (300) im Klimaschrank angeordnet ist.

6. Klimaschrank nach Anspruch 3, wobei die Lagerplätze und die Umschlagvorrichtung (620) gemeinsam auf einer Grundplatte (630) angeordnet sind, welche gegen horizontale Anschläge (651, 652, 653) gedrückt ist, wobei die Anschläge (651, 652, 653) in ihrer Position zur Gerätezentriervorrichtung (151) justiert sind.

7. Klimaschrank nach einem der vorangehenden Ansprüche, wobei die Klimatisiervorrichtung eine Heizvorrichtung (242) und eine Kühlvorrichtung (244) aufweist.

8. Klimaschrank nach Anspruch 7, wobei die Heizvorrichtung (242) und die Kühlvorrichtung (244) als übereinander angeordnete Metallschichten ausgestaltet sind.

## Claims

1. Climate controlled storage cabinet comprising
a climate controlled chamber (2),
a climatization device (306) for generating a given temperature and atmosphere in the climate controlled chamber (2),
a plurality of storage positions (8) arranged in the climate controlled chamber (2) for receiving objects to be stored,
a handling device (620) arranged in the climate controlled chamber (2) for manipulating the objects,
a common controller (300) for the climatization device (306) and the handling device (620),
an auxiliary door (15), which can be opened automatically by the controller (300) and through which objects can be transported into or from the climate controlled chamber,
wherein the climate controlled storage cabinet comprises an object identification device (315) for reading out tags applied to the objects,
**characterized in that** the controller is designed for controlling the climatization device (306), upon opening of the auxiliary door (15) and without waiting for a change in signal of any climate sensors, in such a manner that a heat and/or gas exchange through the opened auxiliary door is counteracted.

2. Climate controlled storage cabinet according to claim 1, wherein the controller comprises an interface (312) for a host-system, via which the climatization device (306) as well as also the handling device (620) are controllable.

3. Climate controlled storage cabinet according to one of the preceding claims, wherein a centering device (151) is designed for a self centering coupling to an external system, particularly to an external object conveyor system.

4. Climate controlled storage cabinet according to one of the preceding claims, wherein the controller (300) is designed for monitoring the storage time of each object in the climate controlled chamber (2).

5. Climate controlled storage cabinet according to one of the preceding claims, wherein the controller (300) is arranged in the climate controlled storage cabinet.

6. Climate controlled storage cabinet according to claim 3, wherein the storage spaces and the handling device (620) are arranged commonly on a base plate (630) which is pressed against horizontal stops (651, 652, 653), wherein the stops (651, 652, 653) are adjusted in their position with respect to the centering device (151).

7. Climate controlled storage cabinet according to one of the preceding claims, wherein the climatization device comprises a heating device (242) and a cooling device (244).

8. Climate controlled storage cabinet according to claim 7, wherein the heating device (242) and the cooling device (244) are designed as metal layers arranged on top of each other.

## Revendications

1. Armoire climatisée comprenant une chambre climatisée (2),
un dispositif de climatisation (306) pour générer une température et une atmosphère prédéterminées dans la chambre climatisée (2),
une pluralité d'emplacements de stockage (8) aménagés dans la chambre climatisée (2) pour recevoir des objets à stocker,
un dispositif de transfert (620) aménagé dans la chambre climatisée (2) pour manipuler les objets,
une commande commune (300) pour le dispositif de climatisation (306) et le dispositif de transfert (620),
une porte auxiliaire (15), qui peut être ouverte automatiquement par la commande (300) et par laquelle des objets peuvent être transportés vers la chambre climatisée ou hors de celle-ci,
dans laquelle l'armoire climatisée présente un dispositif d'identification d'objet (315) pour lire des marques appliquées sur les objets,
**caractérisée en ce que** la commande est conçue pour commander le dispositif de climatisation (306) lors de l'ouverture de la porte auxiliaire (15) et sans attendre une modification de signal d'éventuels capteurs de climatisation de manière à s'opposer à un échange de chaleur et/ou de gaz à travers la porte auxiliaire ouverte.

2. Armoire climatisée selon la revendication 1, dans laquelle la commande présente une interface (312) pour un système hôte, par laquelle peuvent être commandés tant le dispositif de climatisation (306) que le dispositif de transfert (620).

3. Armoire climatisée selon l'une quelconque des revendications précédentes, **caractérisée par** un dispositif de centrage automatique des appareillages (151) conçu pour le couplage, avec centrage automatique, à un système externe, en particulier à un système externe de transport d'objets.

4. Armoire climatisée selon l'une quelconque des revendications précédentes, dans laquelle la commande (300) est conçue pour surveiller le temps de séjour de chaque objet dans la chambre climatisée (2).

5. Armoire climatisée selon l'une quelconque des revendications précédentes, dans laquelle la commande (300) est disposée dans la chambre climatisée.

6. Armoire climatisée selon la revendication 3, dans laquelle les emplacements de stockage et le dispositif de transport (620) sont disposés ensemble sur une plaque de base (630), qui s'appuie contre des butées horizontales (651, 652, 653), lesquelles butées (651, 652, 653) sont ajustées dans leur position par rapport au dispositif de centrage automatique des appareillages (151).

7. Armoire climatisée selon l'une quelconque des revendications précédentes, dans laquelle le dispositif de climatisation présente un dispositif de chauffage (242) et un dispositif de refroidissement (244).

8. Armoire climatisée selon la revendication 7, dans laquelle le dispositif de chauffage (242) et le dispositif de refroidissement (244) se présentent sous la forme de couches métalliques superposées.
